# EUROPÄISCHE PATENTSCHRIFT

(11) **EP 2 024 487 B1**
(45) Veröffentlichungstag und Bekanntmachung des Hinweises auf die Patenterteilung: **31.08.2011**
(21) Anmeldenummer: 07724493.7
(22) Anmeldetag: 24.04.2007
(51) Int. Cl.: C12M 1/00, B01L 3/00

(54) **EINWEGBIOREAKTOR MIT SENSORANORDNUNG**
DISPOSABLE BIOREACTOR COMPRISING A SENSOR ARRANGEMENT
BIORÉACTEUR À USAGE UNIQUE MUNI D'UN SYSTÈME DE DÉTECTION

(30) Priorität: 11.05.2006 DE 102006022307
(43) Veröffentlichungstag der Anmeldung: 18.02.2009
(73) Patentinhaber: Sartorius Stedim Biotech GmbH, 37079 Göttingen (DE); Respironics Novametrix, LLC, Wallingford CT 06492-1942 (US)
(72) Erfinder: BAUMFALK, Reinhard, 37083 Göttingen (DE); REIF, Oscar-Werner, 30173 Hannover (DE); SCHEPER, Thomas, 30559 Hannover (DE); RIECHERS, Daniel, 30451 Hannover (DE); HAYDEN, Russell, Stratford, CT 06614 (US); NUZZO, Philip, Southington, CT 06489 (US)
(74) Vertreter: Schneider, Peter Christian
(86) Internationale Anmeldenummer: PCT/EP2007/003563
(87) Internationale Veröffentlichungsnummer: WO 2007/131593

(56) Entgegenhaltungen:
- WO-A-2005/118771
- WO-A-2005/123258
- WO-A-2006/017951
- US-A1- 2003 023 149
- US-A1- 2005 163 667

## Beschreibung

Die Erfindung bezieht sich auf einen Einwegbioreaktor mit reversibel, äußerlich anbringbarer Sensoranordnung zur Messung einer physikalischen Größe eines enthaltenen Mediums.

Ein derartiger Bioreaktor ist aus der US 2005/0163667 A1 bekannt. Dort wird ein als Beutel ausgebildeter Einwegbioreaktor offenbart, der in seinem Inneren eine Mehrzahl von Sensorkissen aufweist, die fest mit der transparenten Beutelwand verbunden sind. Bei den Sensorkissen handelt es sich um Fluoreszenzsensoren, d.h. Materialien, deren Fluoreszenzeigenschaften sich in Abhängigkeit von bestimmten physikalischen Bedingungen, insbesondere gelöstem Sauerstoff, pH-Wert oder CO₂-Gehalt des im Bioreaktor befindlichen Mediums ändern. Außerhalb der Beutelwand ist im Bereich jedes Sensorkissens eine Detektoranordnung anbringbar. Die Detektoranordnung umfasst jeweils eine Lichtquelle und einen Fotodetektor, der mit einer Auswerteschaltung verbunden ist. Die Lichtquelle strahlt Anregungslicht durch die transparente Beutelwand auf das Sensorkissen, dessen Fluoreszenzlicht von dem Fotodetektor erfasst wird. Mit Hilfe der Auswerteschaltung werden bestimmte Eigenschaften des detektierten Fluoreszenzlichtes analysiert und auf die fotophysikalischen Bedingungen im Sensorkissen, d.h. auf die Größe der zu bestimmenden Werte, geschlossen. Die bekannte Vorrichtung stellt einen Bioreaktor mit Sensorik dar, der bereits herstellerseitig sterilisierbar ist. Einwegbioreaktoren werden üblicherweise mit GammaStrahlung oder sehr aggressiven Chemikalien, wie etwa Ethylenoxid (ETO) sterilisiert. Dabei ergibt sich oft das Problem, dass die Sensorik, insbesondere die Sensorelektronik einen derartigen Sterilisierungsschritt nicht übersteht. Bei der bekannten Vorrichtung wird die Sensorik daher aufgespalten in einen das Medium im Inneren des Bioreaktors berührenden, gegen Sterilisierung robusten Anteil und einen äußerlich anbringbaren, empfindlicheren Teil, insbesondere die Sensorelektronik, der benutzerseitig unsteril angebracht werden kann.

Nachteilig bei der bekannten Vorrichtung ist, dass sie auf das Medium berührenden Sensorkissen basiert. Diese Sensorkissen müssen - wie zuvor erläutert - gegen die Sterilisierung durch Gammastrahlung oder Chemikalien wie ETO resistent sein. D.h. eine solche Behandlung darf ihre Fluoreszenzeigenschaften und insbesondere deren Abhängigkeit von den zu messenden Größen nicht beeinträchtigen. Dies stellt sehr enge Grenzen für die Auswahl der Sensorkissen dar. Anderseits besteht eine weitere Limitierung dergestalt, dass die Sensorkissen ihrerseits das Medium im Bioreaktor nicht beeinflussen dürfen. Insbesondere bei der Verwendung des Einwegbioreaktors als Zellkulturgefäß besteht die Gefahr einer zu innigen Wechselwirkung zwischen den Zellen und den Sensorkissen.

Es ist die Aufgabe der vorliegenden Erfindung einen gattungsgemäßen Einwegbioreaktor derart weiterzubilden, dass die Wechselwirkung zwischen Sensor und Medium minimiert wird.

Diese Aufgabe wird in Verbindung mit den Merkmalen des Oberbegriffs von Anspruch 1 dadurch gelöst, dass in wenigstens einer dem Zu- und/oder Abfluss von Medium dienenden Peripherieleitung des Bioreaktors ein Sensoradapter zur Aufnahme einer über eine innere Grenzfläche des Sensoradapters mit die Peripherieleitung durchströmendem Medium wechselwirkenden, elektronischen Sensoranordnung integriert ist.

Die Erfindung verbindet zwei grundlegende Merkmale. Zum einen liegt der Messort, anders als beim Stand der Technik, nicht im Bereich der Reaktorwand sondern im Bereich einer Peripherieleitung des Bioreaktors. Dies hat den Vorteil, dass eine Wechselwirkung zwischen Sensor und Medium nur während der vergleichsweise kurzen Zeit des Durchstroms von Medium durch die Peripherieleitung erfolgt. Zum anderen vermeidet die vorliegende Erfindung eine unmittelbare Wechselwirkung zwischen dem Sensor und dem Medium. Vielmehr ist vorgesehen, dass die Datenerfassung durch eine Grenzfläche des Sensoradapters hindurch erfolgt, wobei die Grenzfläche so gestaltet sein kann, dass nur eine minimale Wechselwirkung zwischen ihr und dem Medium erfolgt. Der erfindungsgemäße Sensoradapter ist in einen mit dem Einwegreaktor sterilisierbaren Bereich der Peripherieleitung integriert. Er umfasst im Wesentlichen eine Fortsetzung oder einen Einsatz der Peripherieleitung mit Leitungsgrenzflächen aus geeignetem Material, d.h. Material, welches Gamma- bzw. ETO-Sterilisierung übersteht und für die zu messende physikalische Größe bzw. die entsprechenden Sensormittel durchlässig ist. Weiter umfasst der Sensoradapter Anschlussmittel für eine auf die zu messende Größe abgestimmte, elektronische Sensoranordnung. Die Anschlussmittel umfassen dabei vorzugsweise Ausrichtungsmittel, so dass die Sensoranordnung leicht, z.B. durch Einklipsen oder Einschieben in den Sensoradapter, an diesem befestigt werden kann.

Bei einer bevorzugten Ausführungsform der Erfindung ist der Sensoradapter in eine Abgasleitung integriert. Grundsätzlich ist die Erfindung nicht auf eine Zu- oder Abflussleitung und nicht auf ein flüssiges oder gasförmiges Medium beschränkt. Besonders günstig ist es jedoch, den Sensoradapter in eine Peripherieleitung zu integrieren, die der Abführung gasförmigen Mediums dient. Insbesondere bei der Verwendung des Einwegbioreaktors als Zellkulturgefäß stellt die chemische Zusammensetzung des Abgases einen sehr guten Indikator für die Bedingungen in der Zellkultur dar. Insbesondere der CO₂-Gehal des Abgases erlaubt Rückschlüsse auf den Gesundheitszustand der Zellkultur.

Bei einer bevorzugten Ausführungsform der Erfindung ist die Sensoranordnung eingerichtet, durch die innere Grenzfläche hindurch direkt mit dem Medium zu wechselwirken. Insbesondere kann vorgesehen sein, dass die Sensoranordnung einen Infrarotsender zum Aussenden eines infraroten Lichtes durch die Grenzfläche hindurch in das Medium sowie einen, Infrarotdetektor zum Erfassen von Teilen des von dem Infrarotsensor ausgesandten Lichtes nach Wechselwirkung mit dem Medium umfasst. So kann beispielsweise der oben bereits erwähnte CO₂-Gehalt des Abgases anhand der InfrarotAbsorption und/oder -streuung ermittelt werden. Hierbei handelt es sich um eine direkte Wechselwirkung der Sensoranordnung durch die Grenzfläche hindurch mit dem Medium. Dabei ist die Grenzfläche vorzugsweise eine im infraroten Spektralbereich transparente Glas-, Kristall-, oder Kunststofffläche. Insbesondere bei Wahl einer Kunststofffläche ist auf die bereits erwähnte, erforderliche Resistenz gegen die vorgesehene Sterilisierung zu achten.

Alternativ zur direkten Messung durch die Grenzfläche hindurch kann bei einer ebenfalls günstigen Ausführungsform der Erfindung vorgesehen sein, dass die Messung durch indirekte Wechselwirkung zwischen der Sensoranordnung und dem Medium unter Mitwirkung der Grenzfläche erfolgt. Beispielhaft hierfür ist eine Ausführungsform, bei der die Sensoranordnung einen die Temperatur der Grenzfläche erfassenden Temperaturfühler umfasst. Hierbei würde durch Wechselwirkung des Mediums mit der Grenzfläche deren Temperatur verändert und von der Sensoranordnung erfasst. Dabei ist es günstig, wenn die Grenzfläche aus einem mediendichten, wärmeleitenden Material besteht, wobei auch hier auf die Sterilisierungsresistenz zu achten ist.

Als weiteres Beispiel einer indirekten Messung unter Mitwirkung der Grenzfläche kann bei einer alternativen Ausführungsform vorgesehen sein, dass die Sensoranordnung eine Spannungsquelle und zwei mit dieser verbundene Elektroden sowie eine Messanordnung zur Erfassung eines Stroms und/oder einer Spannung zwischen den Elektroden umfasst und dass die Grenzfläche zwei jeweils elektrisch leitfähige, gegeneinander isolierte Teilflächen umfasst, wobei jede der Elektroden mit jeweils einer der Teilflächen in elektrisch leitendem Kontakt steht. Dies schließt den Fall ein, dass die Grenzflächen selbst die Elektroden bilden. Mit einer solchen Ausführungsform kann beispielsweise die Leitfähigkeit des die Peripherieleitung durchströmenden Mediums gemessen werden. Auch hier ist die Grenzfläche selbst in die Messung mit einbezogen. Selbstverständlich ist auch hier auf die Sterilisierungsresistenz des Grenzflächenmaterials zu achten.

Für alle vorgenannten Ausführungsformen gilt, dass eine über die Messung hinausgehende Wechselwirkung zwischen der Grenzfläche und dem Medium, etwa eine chemische Reaktion oder eine Abgabe von Partikeln in das Medium möglichst unterbunden wird.

Weitere Merkmale und Vorteile der Erfindung ergeben sich aus der nachfolgenden speziellen Beschreibung in Verbindung mit den Zeichnungen.

Es zeigen
- Figur 1: eine schematische Darstellung einer bevorzugten Ausführungsform eines erfindungsgemäßen Einwegbioreaktors mit Sensoradapter im sterilen Bereich einer Entgasungsleitung,
- Figur 2: eine schematische Darstellung des Einwegbioreaktors von Figur 1 mit alternativen Anordnungsvorschlägen für den Sensoradapter,
- Figur 3: eine schematische Detailansicht eines für optische Messungen vorgesehenen Sensoradapters,
- Figur 4: eine schematische Detailansicht eines für Temperaturmessungen vorgesehenen Sensoradapters,
- Figur 5: eine schematische Detailansicht eines für Leitfähigkeitsmessungen vorgesehenen Sensoradapters,
- Figur 6: eine schematische Darstellung einer automatisierten periodischen Ansteuerung mehrerer Sensoradapter durch eine gemeinsame Sensorvorrichtung.

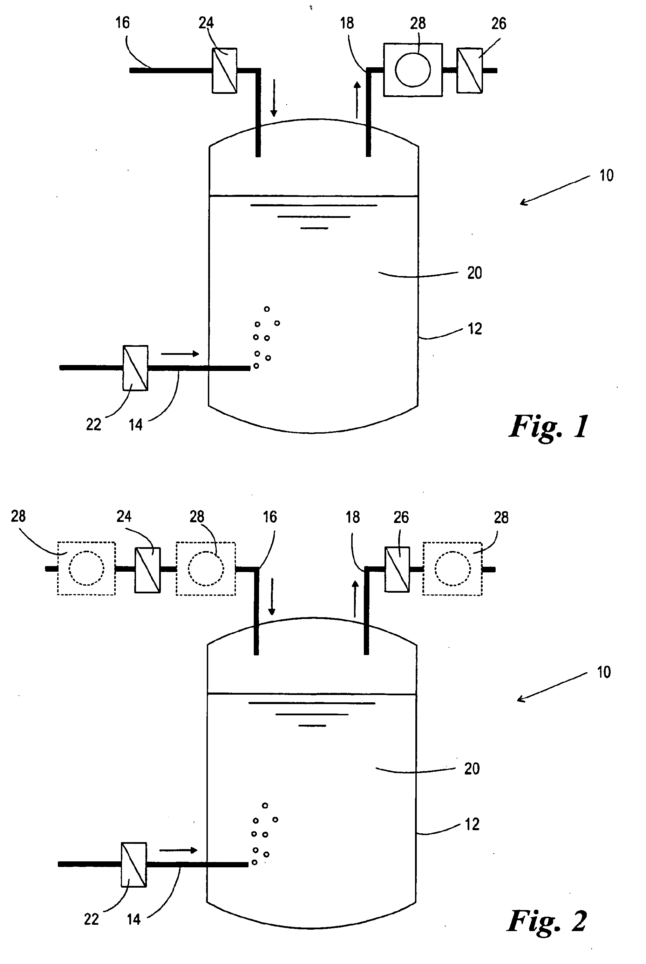

Figur 1 zeigt eine schematische Darstellung eines erfindungsgemäßen Einwegbioreaktors 10. Der Einwegbioreaktor 10 ist vorzugsweise als faltbarer Beutel mit einer Beutelwandung 12 und fest mit dieser verbundenen Peripherieleitungen 14, 16 und 18 ausgestaltet. Die Peripherieleitung 14 ist eine Begasungsleitung, über die Gas in ein in dem Beutel 12 enthaltenes flüssiges Medium 20 eingeleitet wird. Die Peripherieleitung 16 ist eine Zustromleitung für flüssiges und/oder gasförmiges Medium und die Peripherieleitung 18 ist eine Entgasungsleitung. Weitere, in Figur 1 nicht dargestellte Peripherieleitungen zur Zu- oder Abführung flüssigen oder gasförmigen Mediums können ebenfalls vorgesehen sein.

Bei der in Figur 1 dargestellten Ausführungsform sind alle Peripherieleitungen 14, 16 und 18 mit je einem Sterilfilter 22, 24 bzw. 26 versehen. Die Sterilfilter 22, 24 und 26 stellen sicher, dass der steril an den Benutzer ausgelieferte Einwegbioreaktor 10 nicht unbeabsichtigt kontaminiert wird.

Bei der in Figur 1 dargestellten Ausführungsform ist in die Peripherieleitung 18 vor dem Sterilfilter 26 ein Sensoradapter 28 integriert. Der Sensoradapter 28 stellt einen Einsatz in der Peripherieleitung 18 dar, d.h. das abströmende Gas kann durch den Sensoradapter 28 hindurchströmen. Der Sensoradapter 28 ist aus einem Material gefertigt, welches resistent ist gegen die erforderliche Sterilisierung des Bioreaktors 10, die anwendungsspezifisch variieren kann. Besonders günstige Sterilisierungsverfahren sind die Gamma-Sterilisierung sowie die chemische Sterilisierung mit ETO. Weiter ist die Materialwahl des Sensoradapters auf die spezielle, zu adaptierende Sensorik abgestimmt. Beispiele hierzu werden weiter unten erläutert.

Figur 2 zeigt eine schematische Darstellung des Bioreaktors von Figur 1 mit weiteren Anbringungsmöglichkeiten für Sensoradapter 28. Zur Veranschaulichung der Optionalität der Anordnungen sind die Sensoradapter 28 in Figur 2 gestrichelt dargestellt. Die im Einzelfall realisierte Anordnung des Sensoradapters ist vom Fachmann an die zu messende Größe im Hinblick auf deren Zugänglichkeit zu wählen. Ausführungsformen mit mehreren Sensoradaptern, die gleich oder unterschiedlich ausgebildet sein können, sind denkbar.

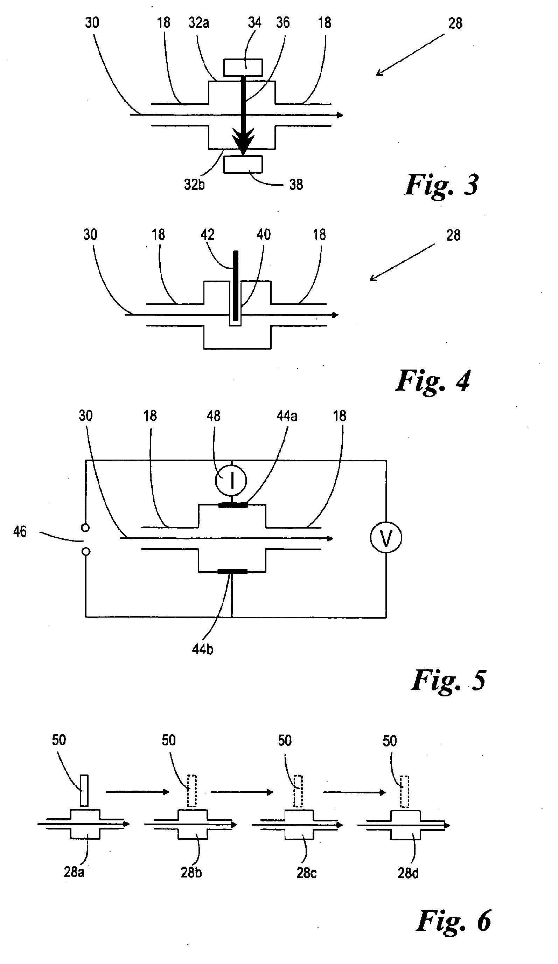

Figur 3 zeigt eine besondere Ausführungsform eines Sensoradapters 28 für optische Messungen. Der Sensoradapter 28 wird, wie durch den Pfeil 30 angezeigt, von einem Medium, insbesondere dem Abgas, welches durch die Peripherieleitung 18 fließt, durchströmt. Der Sensoradapter weist ein Paar gegenüberliegender Fenster 32a und 32b auf, die für infrarote Strahlung transparent sind. Ein Infrarotsender 34 sendet einen Infrarotstrahl 36 durch das Eingangsfenster 32a, das den Sensoradapter durchströmende Medium 30 und das Ausgangsfenster 32b. Der Infrarotstrahl 36 wird von einem Infrarotdetektor 38 empfangen, der mit einer in Figur 3 nicht dargestellten Auswerteeinheit verbunden ist. Durch Auswertung optischer Eigenschaften des empfangenen Strahls 36 kann dann auf Eigenschaften des durchströmenden Mediums 30 geschlossen werden. Insbesondere zur Messung des CO₂-Gahltes im Abgas einer in dem Einwegbioreaktor kultivierten Zellkultur ist dieses Verfahren der IR-Analyse des Gases vorteilhaft. Die physikalischen Einzelheiten einer solchen Messung sind aus der Medizin im Bereich der Atemluftanalyse bekannt.

Figur 4 zeigt eine alternative Ausführungsform eines Sensoradapters 28. Der Sensoradapter weist eine Vertiefung auf, die von einem wärmeleitfähigen Wandabschnitt umgeben ist und in das Innere des Durchströmungsvolumens hineinreicht, so dass der Wandabschnitt 40 von dem den Sensoradapter 28 durchströmendem Gas 30 umströmt wird. Hierbei kommt es zu einem Wärmeaustausch zwischen dem Gas und dem Wandbereich 40. Mittels eines Temperatursensors 42 kann die Temperatur des Wandbereichs 40 erfasst und auf die Temperatur des Gases geschlossen werden.

Figur 5 zeigt eine weitere Ausführungsform eines Sensoradapters, der hier zur Messung der Leitfähigkeit des durchströmenden Mediums 30 einsetzbar ist. Der Sensoradapter 28 weist elektrisch leitfähige und elektrisch gegeneinander isolierte Wandbereiche 44a und 44b auf, die mit einer Spannungsversorgung 46 und einer Strommessanordnung 48 verbunden sind. Zwischen den als Elektroden wirkenden Wandbereichen 44a und 44b kann bei Durchströmung mit einem elektrisch leitfähigen Medium ein elektrischer Strom fließen, dessen Messung Rückschlüsse auf die elektrische Leitfähigkeit des Mediums 30 erlaubt.

Figur 6 zeigt eine besonders vorteilhafte Anwendung des erfindungsgemäßen Einwegbioreaktors mit Sensoradapter. Repräsentativ für verschiedene Einwegbioreaktoren sind in Figur 6 deren Sensoradapter 28a, 28b, 28c und 28d dargestellt. Ein einzelner Sensor, der beispielsweise ein optischer Sensor entsprechend Figur 3, ein Wärmesensor entsprechend Figur 4, ein Leitfähigkeitssensor entsprechend Figur 5 oder ein anderer auf die Sensoradapter 28a, 28b, 28c und 28d abgestimmter Sensor sein kann, wird vorzugsweise automatisiert nacheinander an jede der Sensoradapter 28a, 28b, 28c und 28d angelegt, um eine Messung durchzuführen, und anschließend zum nächsten Sensoradapter weiterbewegt. Dies ist möglich ohne Kontaminationsgefahr des Einwegbioreaktors, da der Sensor 50 nur mit dem Äußeren bzw. mit den vorgesehenen Koppelstellen der Sensoradapter in Berührung kommt. Um dies erleichtern, können bei den Sensoradaptern in den Figuren nicht dargestellte Ausrichtungs- und Koppelmittel vorgesehen sein, wie beispielsweise Rastvorrichtungen, Zentrierhilfen und/oder Schienensysteme.

Natürlich stellen die in der speziellen Beschreibung diskutierten und in den Figuren veranschaulichten Ausführungsformen nur illustrative Ausführungsbeispiele der vorliegenden Erfindung dar. Dem Fachmann ist ein weites Spektrum an Modifikationsmöglichkeiten anhand gegeben. Insbesondere die Material- und Formenwahl ist auf die spezielle Anwendung, die vorgesehenen Sterilisierungsverfahren und die einzusetzenden Messprinzipien abzustimmen.

## Patentansprüche

1. Einwegbioreaktor mit reversibel, äußerlich anbringbarer Sensoranordnung zur Messung einer physikalischen Größe eines enthaltenen Mediums, **dadurch gekennzeichnet,**
**dass** in wenigstens einer dem Zu- und/oder Abfluss von Medium dienenden Peripherieleitung (14, 16, 18) des Bioreaktors ein Sensoradapter (28) zur Aufnahme einer über eine innere Grenzfläche (32a, 32b; 40; 44a, 44b) des Sensoradapters mit die Peripherieleitung (14, 16, 18) durchströmendem Medium wechselwirkenden, elektronischen Sensoranordnung (34, 38; 42; 44a, 44b, 46, 48) integriert ist und dass der Sensoradapter (28) als ein die Peripherieleitung (14, 16, 18) fortsetzender Einsatz, der von dem enthaltenen Medium durchströmbar ist, in der Peripherieleitung (14, 16, 18) ausgeführt ist.

2. Einwegbioreaktor nach Anspruch 1,
**dadurch gekennzeichnet,**
**dass** der Sensoradapter (28) in eine Abgasleitung (18) integriert ist.

3. Einwegbioreaktor nach einem der vorangehenden Ansprüche,
**dadurch gekennzeichnet,**
**dass** die Sensoranordnung (34, 38; 41; 44a, 44b, 46, 48) eingerichtet ist, durch die innere Grenzfläche (32a, 32b; 40; 44a, 44b) hindurch direkt mit dem Medium zu wechselwirken.

4. Einwegbioreaktor nach einem der vorangehenden Ansprüche,
**dadurch gekennzeichnet,**
**dass** die Sensoranordnung einen Infrarotsender (34) zum Aussenden eines infraroten Lichtes durch die Grenzfläche (32a, 32b) hindurch in das Medium sowie einen Infrarotdetektor (38) zum Erfassen von Teilen des von dem Infrarotsensor (34) ausgesandten Lichtes nach Wechselwirkung mit dem Medium umfasst.

5. Einwegbioreaktor nach Anspruch 4,
**dadurch gekennzeichnet,**
**dass** die Grenzfläche (32a, 32b) eine im infraroten Spektralbereich transparente Glas-, Kristall- oder Kunststofffläche ist.

6. Einwegbioreaktor nach einem der Ansprüche 1 bis 3,
**dadurch gekennzeichnet,**
**dass** die Sensoranordnung einen die Temperatur der Grenzfläche (40) erfassenden Temperaturfühler (42) umfasst.

7. Einwegbioreaktor nach Anspruch 6,
**dadurch gekennzeichnet,**
**dass** die Grenzfläche (40) aus einem mediendichten, wärmeleitfähigen Material besteht.

8. Einwegbioreaktor nach einem der Ansprüche 1 bis 3,
**dadurch gekennzeichnet,**
**dass** die Sensoranordnung eine Spannungsquelle (46) und zwei mit dieser verbundene Elektroden (44a, 44b) sowie eine Messanordnung (48) zur Erfassung eines Stroms und/oder einer Spannung zwischen den Elektroden (44a, 44b) umfasst und dass die Grenzfläche zwei jeweils elektrisch leitfähige, gegeneinander isolierte Teilflächen umfasst, wobei jede der Elektroden (44a, 44b) mit jeweils einer der Teilflächen in elektrisch leitendem Kontakt steht.

9. Einwegbioreaktor nach einem der vorangehenden Ansprüche,
**dadurch gekennzeichnet,**
**dass** der Sensoradapter (28) aus einem gegen Gammastrahlung resistenten Material besteht.

10. Einwegbioreaktor nach einem der vorangehenden Ansprüche,
**dadurch gekennzeichnet,**
**dass** der Sensoradapter (28) aus einem gegen ETO resistenten Material besteht.

## Claims

1. A disposable bioreactor comprising a sensor arrangement, which can be reversibly fitted on the outside, for measuring a physical variable of a contained medium,
**characterized in that**
there is integrated, in at least one peripheral line (14, 16, 18) of the bioreactor serving to supply and/or discharge medium, a sensor adaptor (28) for holding an electronic sensor arrangement (34, 38; 42; 44a, 44b, 46, 48) interacting, via an internal boundary surface (32a, 32b; 40; 44b) of the sensor adaptor, with the medium flowing through the peripheral line (14, 16, 18), and **in that** the sensor adaptor (28) is embodied in the peripheral line (14, 16, 18) as an insert that extends the peripheral line (14, 16, 18) and through which the contained medium can flow.

2. The disposable bioreactor according to Claim 1,
**characterized in that**
the sensor adaptor (28) is integrated in an exhaust gas line (18).

3. The disposable bioreactor according to any of the preceding claims,
**characterized in that**
the sensor arrangement (34, 38; 41; 44a, 44b, 46, 48) is set up to interact directly with the medium through the internal boundary surface (32a, 32b; 40; 44a, 44b).

4. The disposable bioreactor according to any of the preceding claims,
**characterized in that**
the sensor arrangement comprises an infrared transmitter (34) for emitting an infrared light through the boundary surface (32a, 32b) into the medium, and an infrared detector (38) for detecting portions of the light emitted by the infrared transmitter (34) after interaction with the medium.

5. The disposable bioreactor according to Claim 4,
**characterized in that**
the boundary surface (32a, 32b) is a glass, crystal or plastic surface transparent in the infrared spectral region.

6. The disposable bioreactor according to any of the Claims 1 to 3,
**characterized in that**
the sensor arrangement comprises a temperature detector (42) that detects the temperature of the boundary surface (40).

7. The disposable bioreactor according to Claim 6, **characterized in that** the boundary surface (40) consists of a medium-tight, thermally conducting material.

8. The disposable bioreactor according to any of the Claims 1 to 3,
**characterized in that**
the sensor arrangement comprises a voltage source (46) and two electrodes (44a, 44b) connected thereto, and a measuring arrangement (48) for detecting a current and/or a voltage between the electrodes (44a, 44b), and **in that** the boundary surface comprises two electrically conducting, mutually insulated subsurfaces, each of the electrodes (44a, 44b) being in electrically conducting contact with in each case one of the subsurfaces.

9. The disposable bioreactor according to any of the preceding claims,
**characterized in that**
the sensor adaptor (28) consists of a material resistant to gamma radiation.

10. The disposable bioreactor according to any of the preceding claims,
**characterized in that**
the sensor adaptor (28) consists of a material resistant to ETO.

## Revendications

1. Bioréacteur à usage unique qui comprend un agencement capteur pouvant être monté de manière réversible à l'extérieur, pour mesurer une variable physique d'un milieu contenu, **caractérisé en ce qu'**un adaptateur de capteur (28) est intégré dans au moins une conduite périphérique (14, 16, 18) du bioréacteur servant à alimenter et/ou décharger le milieu, pour recevoir un agencement capteur électronique (34, 38; 42; 44a, 44b, 46, 48) en interaction, via une surface-limite (32a, 32b; 40; 44b) de l'adaptateur de capteur, avec le milieu parcourant la conduite périphérique (14, 16, 18), et **en ce que** l'adaptateur de capteur (28) est monté dans la conduite périphérique (14, 16, 18) sous forme d'un insert qui prolonge la conduite périphérique (14, 16, 18) et par lequel le milieu contenu peut couler.

2. Bioréacteur à usage unique selon la revendication 1, **caractérisé en ce que** l'adaptateur de capteur (28) est intégré dans un conduit d'échappement de gaz (18).

3. Bioréacteur à usage unique selon l'une quelconque des revendications précédentes, **caractérisé en ce que** l'agencement capteur (34, 38; 41; 44a, 44b, 46, 48) est configuré pour interagir directement avec le milieu à travers la surface-limite intérieure (32a, 32b; 40; 44b).

4. Bioréacteur à usage unique selon l'une quelconque des revendications précédentes, **caractérisé en ce que** l'agencement capteur comprend un émetteur infrarouge (34) pour émettre une lumière infrarouge à travers la surface-limite intérieure (32a, 32b) dans le milieu, et un capteur infrarouge (38) pour détecter des parties de lumière émise par l'émetteur infrarouge (34) après interaction avec le milieu.

5. Bioréacteur à usage unique selon la revendication 4, **caractérisé en ce que** la surface-limite (32a, 32b) est une surface en verre, cristal ou plastique transparente dans la région spectrale infrarouge.

6. Bioréacteur à usage unique selon l'une quelconque des revendications 1 à 3, **caractérisé en ce que** l'agencement capteur comprend un capteur de température (42) qui détecte la température de la surface-limite (40).

7. Bioréacteur à usage unique selon la revendication 6, **caractérisé en ce que** la surface-limite (40) est constituée d'un matériau thermiquement conducteur et étanche par rapport au milieu.

8. Bioréacteur à usage unique selon l'une quelconque des revendications 1 à 3, **caractérisé en ce que** l'agencement capteur comprend une source de tension (46) et deux électrodes (44a, 44b) branchées à ladite source de tension, et un dispositif de mesure (48) pour détecter un courant et/ou une tension entre les électrodes (44a, 44b), et **en ce que** la surface-limite comprend deux sous-surfaces conductrices et mutuellement isolées, chacune des électrodes (44a, 44b) étant en contact conducteur avec, dans chaque cas, l'une des sous-surfaces.

9. Bioréacteur à usage unique selon une quelconque des revendications précédentes, **caractérisé en ce que** l'adaptateur de capteur (28) est constitué d'un matériau résistant au rayonnement gamma.

10. Bioréacteur à usage unique selon une quelconque des revendications précédentes, **caractérisé en ce que** l'adaptateur de capteur (28) est constitué d'un matériau résistant à l'oxyde d'éthylène.
